# EUROPEAN PATENT SPECIFICATION

(11) **EP 3 037 422 B1**
(45) Date of publication and mention of the grant of the patent: **13.09.2017**
(21) Application number: 14199990.4
(22) Date of filing: 23.12.2014
(51) Int. Cl.: C07D 487/04, C07D 207/32, C07D 211/80, H01L 51/00

(54) **Condensed-cyclic compound and organic light emitting diode having organic layer including the same**
Kondensiert-cyclische Verbindung und organische lichtemittierende Diode mit organischer Schicht damit
Composés cycliques condensés et diode électroluminescente organique dotée d'une couche organique les incluant

(43) Date of publication of application: 29.06.2016
(73) Proprietor: Samsung Display Co., Ltd., Gyeonggi-do (KR)
(72) Inventor: Lee, Kwan-Hee, 446-711 Yongin-City (KR); Kim, Mi-Kyung, 446-711 Yongin-City (KR); Yang, Seung-Gak, 446-711 Yongin-City (KR)
(74) Representative: Gulde & Partner

(56) References cited:
- WO-A1-2013/050862
- KR-A- 20130 096 647
- US-A1- 2014 367 654

## Description

### BACKGROUND

### Field

A condensed-cyclic compound and an organic light emitting diode having an organic layer including the condensed-cyclic are provided.

### Description of the Related Art

Organic light emitting diodes are self-emission devices, have wide viewing angles, high contrast ratios, short response time, high luminosity, low driving voltages, high response rates, and produce various colors.

A conventional organic light emitting diode may have the following structure. An anode is formed on a substrate, and then a hole transport layer, a light emitting layer, an electron transport layer, and a cathode are sequentially formed on the anode. In this regard, each of the hole transport layer, the light emitting layer, and the electron transport layer is an organic thin film formed of an organic compound.

An organic light emitting diode having the structure described above may have the following driving principle.

When a voltage is applied between the anode and the cathode, holes injected from the anode move to the light emitting layer through the hole transport layer, and electrons injected from the cathode move to the light emitting layer through the electron transport layer. The holes and electrons, which are carriers, are recombined in the light emitting layer to form excitons. These excitons are changed from an excited state to a ground state, thereby generating light.

US 2014/367654 A1 and WO 2013/050862 Aldisclose exemplary condensed-cyclic compounds used in organic electroluminescent elements.

### SUMMARY

The present invention provides a compound capable of providing an organic light emitting diode having a low driving voltage, a high current density, high efficiency, high quantum efficiency, and high luminosity.

According to an aspect of the present invention, there is provided a condensed-cyclic compound represented by one of:
Compound 1-1: 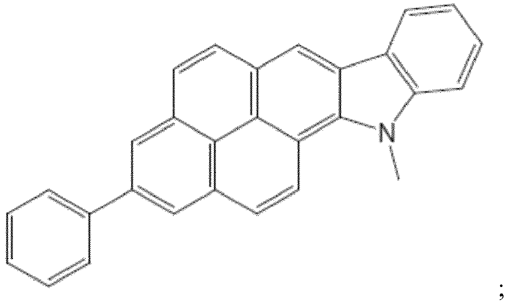
Compound 1-2: 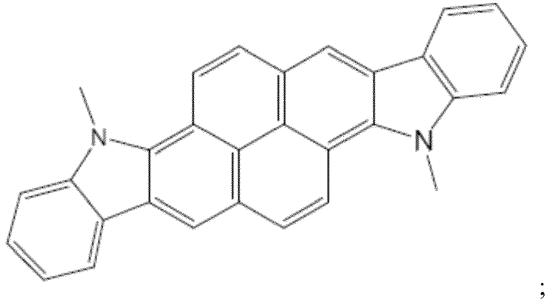
Compound 1-3:

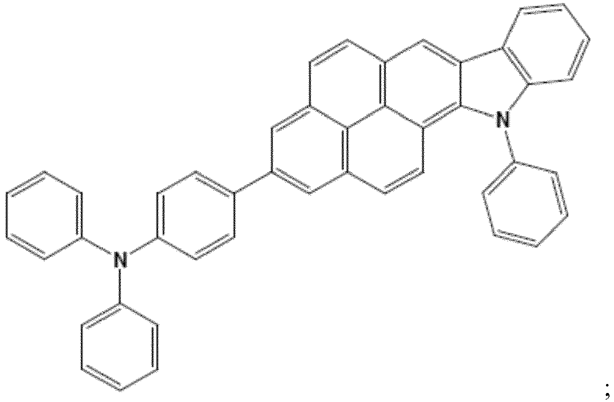

According to another aspect of the present invention, there is provided an organic light emitting diode comprising: a first electrode, a second electrode facing the first electrode, and an organic layer interposed between the first electrode and the second electrode, wherein the organic layer comprises the condensed-cyclic compound of the present invention. The organic layer may comprise a light emitting layer.

### BRIEF DESCRIPTION OF THE DRAWINGS

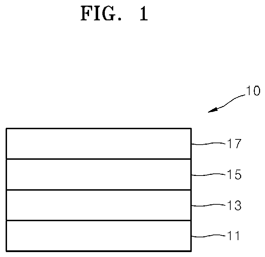

The above and other features and advantages of embodiments of the present invention will become more apparent by describing in detail exemplary embodiments thereof with reference to FIG. 1 showing a schematic view of an organic light emitting diode according to an embodiment.

### DETAILED DESCRIPTION

The condensed-cyclic compound according to the present invention is represented by one of Compound 1-1, Compound 1-2, and/or Compound 1-3, wherein:
Compound 1-1 is: 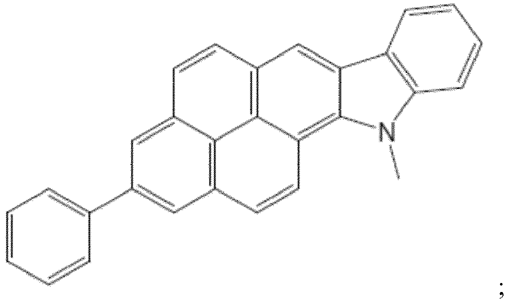
Compound 1-2 is: 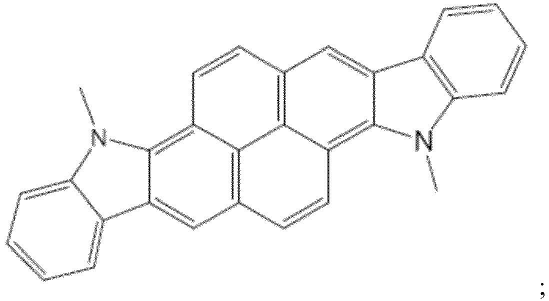
Compound 1-3 is:

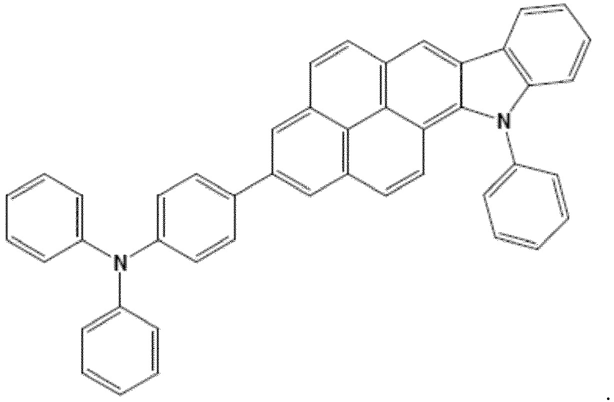

The organic light emitting diode employing one of the condensed-cyclic compounds Compound 1-1, Compound 1-2, and/or Compound 1-3 exhibits low driving voltage, high current density, high luminosity, high efficiency, and long lifetime.

The condensed-cyclic compound of the present invention represented by Compound 1-1, Compound 1-2, and Compound 1-3 can be synthesized using known organic synthesis method. A method of synthesizing the condensed-cyclic compound may be obvious to one of ordinary skill in the art by referring to the prior art of record, in particular in EP application with the publication number EP 2 292 604.

The condensed-cyclic compound of the present invention may be included in an organic layer of an organic light emitting diode. Thus, an organic light emitting diode including a first electrode, a second electrode facing the first electrode, and an organic layer interposed between the first electrode and the second electrode, wherein the organic layer includes the condensed-cyclic compound of Formula 1, is provided.

The organic layer may be a light emitting layer or a hole transport layer.

For example, when the organic layer is the light emitting layer, the light emitting layer may further include, in addition to the condensed-cyclic compound represented by Compound 1-1, Compound 1-2, and/or Compound 1-3, a known host. However, the organic layer may also include other materials in another embodiment.

FIG. 1 is a schematic view of an organic light emitting diode 10 according to an embodiment of the present invention. Hereinafter, the structure of the organic light emitting diode 10 and a method of manufacturing the organic light emitting diode 10 will be described.

The organic light emitting diode 10 includes a substrate 11, a first electrode 13, an organic layer 15, and a second electrode 17 sequentially formed in this stated order.

The substrate 11 may be any substrate that is used in a commonly used organic light emitting diode. For example, the substrate 11 may be a glass substrate or a transparent plastic substrate, either of which has high mechanical strength, high thermal stability, high transparency, surface smoothness and is waterproof.

The first electrode 13 may be formed by depositing or sputtering a first electrode material on the substrate 11. If the first electrode 13 is an anode, the first electrode material may include a material having a high work function constant so that holes are easily injected. The first electrode 13 may be a reflective electrode or a transmissive electrode. The first electrode material may be a material that is transparent and highly conductive, such as indium tin oxide (ITO), indium zinc oxide (IZO), tin oxide (SnO₂), or zinc oxide (ZnO). Alternatively, the first electrode material may be magnesium (Mg), aluminum (Al), aluminum-lithium (Al-Li), potassium (K), magnesium-indium (Mg-In), or magnesium-silver (Mg-Ag).

The organic layer 15 is formed on the first electrode 13. In the present specification, the term "organic layer" indicates the layers interposed between the first electrode 13 and the second electrode 17. For example, the organic layer may also include a metallic complex. For example, the organic layer may further include other materials other than an organic material.

The organic layer 15 may include at least one layer selected from the group consisting of a hole injection layer, a hole transport layer, a light emitting layer, a hole blocking layer, an electron transport layer, and an electron injection layer.

The hole injection layer (HIL) may be formed on the first electrode 13 using a method selected from various known methods such as a vacuum deposition method, a spin coating method, a cast method, or a Langmuir Blodgett (LB) method.

If the HIL is formed using a vacuum deposition method, deposition conditions may differ according to a compound selected for preparing a target layer, a target layer structure, and thermal characteristics, and for example, a deposition temperature may be from about 100 to about 500°C, a degree of vacuum may be from about 10⁻¹⁰ to about 10⁻³ torr, and a deposition speed may be from about 0.01 to about 100 Å/sec, but the deposition temperature, the degree of vacuum, and the deposition speed is not limited thereto.

If the HIL is formed using a spin coating method, coating conditions may differ according to a compound selected for preparing a target layer, a target layer structure, and thermal characteristics, and for example, a coating speed may be from about 2000 rpm to about 5000 rpm and a temperature at which a solvent used is removed after coating may be from about 80°C to about 200°C, but the coating speed and the solvent removal temperature are not limited thereto.

A HIL material may be any known hole injection material and may be, for example, N,N'-diphenyl-N,N'-bis-[4-(phenyl-m-tolyl-amino)-phenyl]-biphenyl-4,4'-diamine (DNTPD) or a phthalocyanine compound such as a copper phthalocyanine, 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), or (polyaniline)/poly(4-styrenesulfonate) (PANI/PSS). Exemplary compounds are shown below:

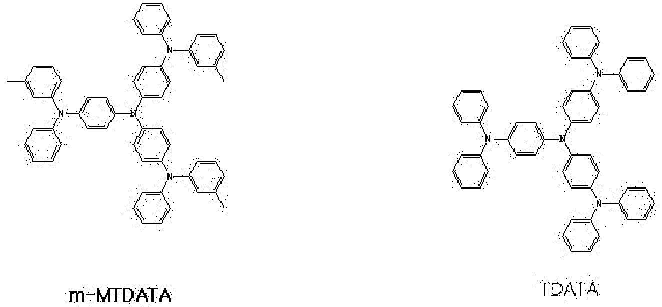

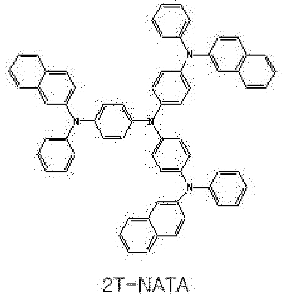

The thickness of the HIL may be from about 100 Å to about 10000 Å, for example, from about 100 Å to about 1000 Å. If the thickness of the HIL is within this range, satisfactory hole injection characteristics may be obtained without a substantial increase in the driving voltage of the organic light emitting diode.

The hole transport layer (HTL) may be formed on the HIL using a method selected from various known methods such as a vacuum deposition method, a spin coating method, a cast method, or a LB method. When the HTL is formed using a vacuum deposition method or a spin coating method, deposition conditions and coating conditions may differ according to a compound selected for preparing a target layer, but may be similar to those described with reference to the HIL.

A HTL material may be any known hole transport material, and may be, for example, a carbazole derivative such as N-phenylcarbazole or polyvinylcarbazole; an amine derivative having an aromatic condensation rin g (such as N,N'-bis(3-methylphenyl)-N,N'-diphenyl-[1,1-biphenyl]-4,4'-diamine (TPD) or N,N'-di(naphthalene-1-yl)-N,N'-diphenyl benzidine (NPD); or a triphenylamine-based material, such as 4,4',4"-tris(N-carbazolyl)triphenylamine (TCTA). Among these HTL materials, TCTA may have, in addition to a hole transporting capability, a capability of blocking diffusion of excitons generated in the light emitting layer. Exemplary compounds are shown below:

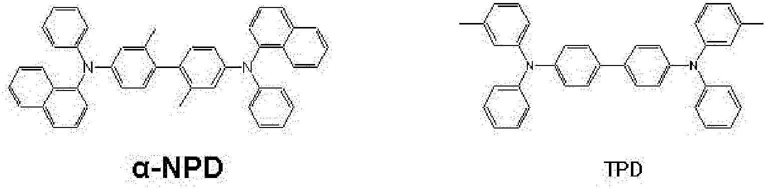

The thickness of the HTL may be from about 50 Å to about 1000 Å, for example, from about 100 Å to about 800 Å. If the thickness of the HTL is within this range, satisfactory hole transporting characteristics may be obtained without a substantial increase in the driving voltage of the organic light emitting diode.

The light emitting layer (EML) may be formed on the HTL using a method selected from various known methods such as a vacuum deposition method, a spin coating method, a cast method, or a LB method. When the EML is formed using a vacuum deposition method or a spin coating method, deposition conditions and coating conditions may differ according to a compound selected for preparing a target layer, but may be similar to those described with reference to the HIL.

The EML may include the condensed-cyclic compound of Formula 1 described above. The EML may include only the condensed-cyclic compound, or further include, in addition to the condensed-cyclic compound, a known host. The known host may be Alq₃, 4,4'-N,N'-dicarbazole-biphenyl (CBP), poly(n-vinylcarbazole) (PVK), 9,10-di(naphthalene-2-yl)anthracene(ADN), TCTA, 1,3,5-tris(N-phenylbenzimidazole-2-yl)benzene (TPBI), 3-tert-butyl-9,10-di(naphth-2-yl) anthracene (TBADN), E3, or distyrylarylene (DSA). Exemplary compounds are shown below:

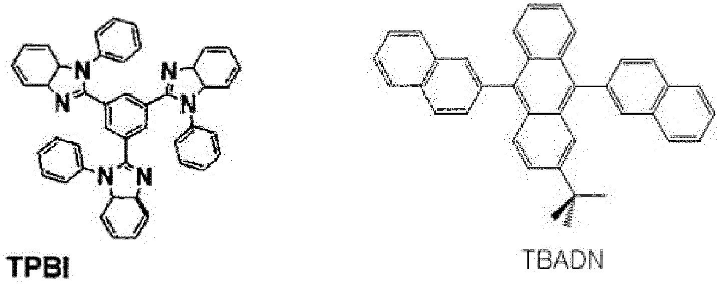

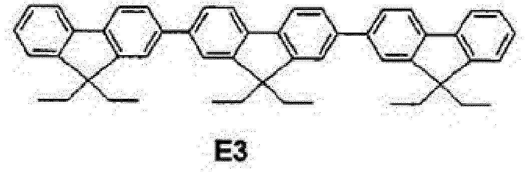

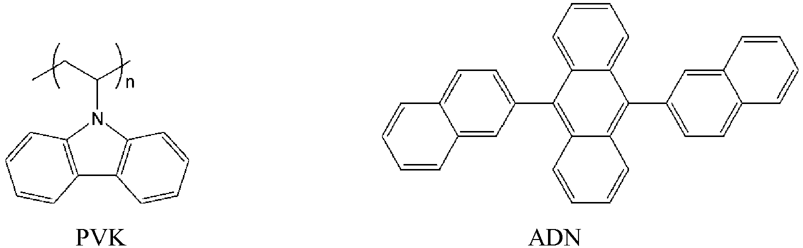

When the condensed-cyclic compound of the present invention (dopant) is used together with a host, the doping concentration of the condensed-cyclic compound may vary. For example, in general, the doping concentration of the condensed-cyclic compound may be from about 0.01 to about 15 parts by weight based on about 100 parts by weight of the host.

The thickness of the EML may be from about 100 Å to about 1000 Å, for example, from about 200 Å to about 600 Å. If the thickness of the EML is within this range, excellent light emitting characteristics may be obtained without a substantial increase in the driving voltage of the organic light emitting diode.

When a phosphorescent dopant is used to form the EML, a hole blocking layer (HBL) may be further formed between the HTL and the EML. The HBL blocks triplet excitons or holes from diffusing into, for example, the ETL. When the HBL is formed using a vacuum deposition method or a spin coating method, deposition conditions and coating conditions may differ according to a compound selected for preparing a target layer, but may be similar to those described with reference to the HIL. A hole blocking material may be any known hole blocking material. For example, the hole blocking material may be an oxadiazole derivative, a triazole derivative, or a phenanthroline derivative.

The thickness of the HBL may be from about 50 Å to about 1000 Å, for example, from about 100 Å to about 300 Å. If the thickness of the HBL is within this range, excellent hole blocking characteristics may be obtained without a substantial increase in the driving voltage of the organic light emitting diode.

Then, electron transport layer (ETL) may be formed using a method selected from various known methods such as a vacuum deposition method, a spin coating method, or a cast method. When the ETL is formed using a vacuum deposition method or a spin coating method, deposition conditions and coating conditions may differ according to a compound selected for preparing a target layer, but may be similar to those described with reference to the HIL. An ETL material may stably transport electrons injected from an electron injection electrode (cathode), and may be any known electron transporting material. For example, the ETL may be a quinoline derivative such as tris(8-quinolinolate)aluminum (Alq₃), TAZ, Balq, or beryllium bis benzoquinolin-10-olate (Bebq₂). Exemplary compounds are shown below:

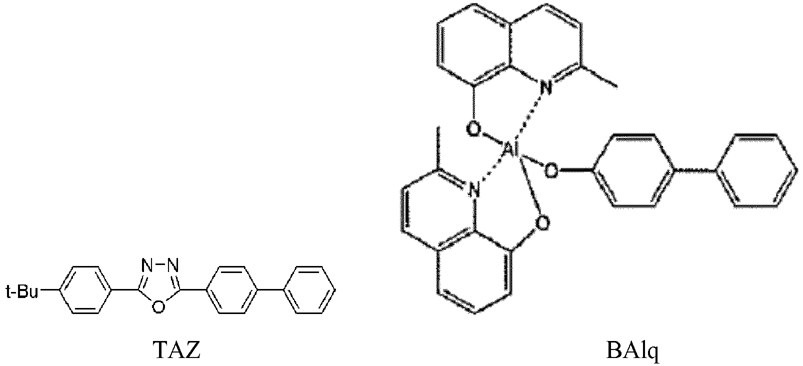

The thickness of the ETL may be from about 100 Å to about 1000 Å, for example, from about 150 Å to about 500 Å. If the thickness of the ETL is within this range, excellent electron transporting characteristics may be obtained without a substantial increase in the driving voltage of the organic light emitting diode.

In addition, the electron injection layer (EIL) may be formed on the ETL. The EIL may include an EIL material that allows electrons to be easily injected from a cathode, and the EIL material may not be particularly limited.

The EIL material may be any known EIL material such as LiF, NaCl, CsF, Li₂O, or BaO. Deposition conditions for forming the EIL may differ according to a compound selected for preparing a target layer, and may be similar to those described with reference to the HIL.

The thickness of the EIL may be from about 1 Å to about 100 Å, for example, from about 3 Å to about 90 Å. If the thickness of the EIL is within this range, excellent electron injection characteristics may be obtained without a substantial increase in the driving voltage of the organic light emitting diode.

The second electrode 17 may be formed on the organic layer 15. The second electrode 17 may be a transmissive electrode. The second electrode 17 may be a cathode that is an electron injection electrode. When the second electrode 17 is a cathode, the second electrode 17 may be manufactured using a metal, an alloy, an electrically conductive compound, or a mixture thereof, each of which has a low work function constant, such as lithium (Li), magnesium (Mg), aluminum (Al), aluminum-lithium (Al-Li), potassium (K), magnesium-indium (Mg-In), or magnesium-silver (Mg-Ag). In addition, for a top emission type light emitting diode, the second electrode 17 may be a transmissive electrode including ITO or IZO.

Hereinafter, an organic light emitting diode according to an embodiment will be described in detail with reference to examples.

### EXAMPLES

### Example 1-1

An ITO layer on a substrate was patterned such that a light emitting area had a size of 2 mm × 2 mm, and then washed. The substrate including the patterned ITO layer was loaded into a vacuum chamber and then, a base pressure was controlled to be 1 × 10-6 torr. Then, a DNTPD layer (700 Å), an NPD layer (300 Å), a layer including AND (host) and Compound 1-1 (dopant: 3 weight%) (250 Å), a Bebq2 layer (250 Å), a LiF layer (5 Å), and an Al layer (700 Å) were sequentially formed on the ITO layer on the substrate in this stated order, thereby manufacturing an organic light emitting diode.

### <Compound 1-1>

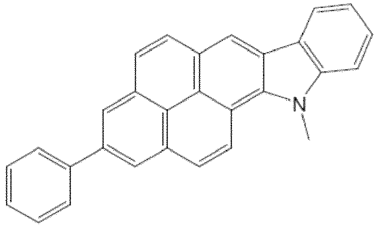

### Example 1-2

An organic light emitting diode was manufactured in the same manner as in Example 1-1, except that Compound 1-2 was used instead of Compound 1-1; with:

### <Compound 1-2>

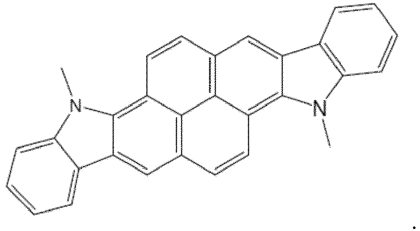

### Example 1-3

An organic light emitting diode was manufactured in the same manner as in Example 1-1, except that Compound 1-3 was used instead of Compound 1-1; with:

### <Compound 1-3>

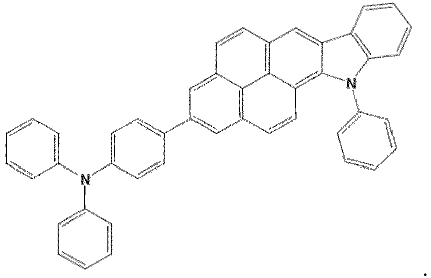

### Example 1-4 (not falling under the present invention)

An organic light emitting diode was manufactured in the same manner as in Example 1-1, except that Compound 1-4 was used instead of Compound 1-1, with:

### <Compound 1-4>

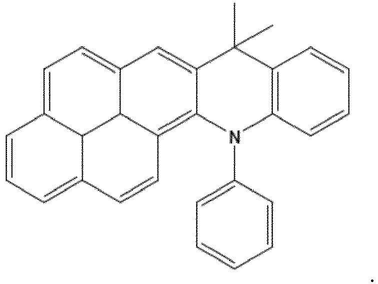

### Example A

An organic light emitting diode was manufactured in the same manner as in Example 1-1, except that Compound A was used instead of Compound 1-1; with:

### <Compound A>

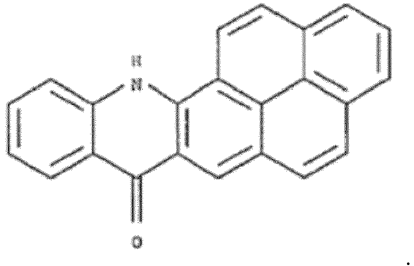

### Example B

An organic light emitting diode was manufactured in the same manner as in Example 1-1, except that Compound B was used instead of Compound 1-1; with:

### <Compound B>

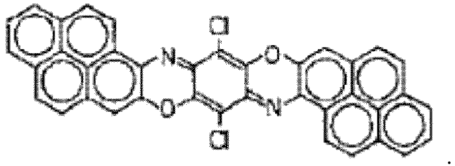

### Evaluation Example

The driving voltage, current at the driving voltage, current density, brightness, electric power, quantum efficiency, and efficiency of the organic light emitting diodes manufactured according to Examples 1-1 to 1-4 and Comparative Examples A and B were evaluated using a PR650 (Spectroscan) Source Measurement Unit. (Manufacturer: PhotoResearch Co., Chatworth, CA). The results are shown in Table 1 below.

**Table 1**

| | Example 1-1 | Example 1-2 | Example 1-3 | Example 1-4 | Example A | Example B |
|---|---|---|---|---|---|---|
| Current density (mA/cm²) | 10 | 10 | 10 | 10 | 10 | 10 |
| Brightness (Cd/A) | 7.71 | 8.01 | 9.42 | 7.32 | 1.78 | 1.85 |
| Electric power (Im/W) | 4.8 | 4.5 | 6.2 | 4.1 | 1.02 | 1.09 |
| Efficiency (Cd/m²) (magnification) | 810 | 753 | 922 | 704 | 178 | 185 |

From the Table 1, it is clear that the brightness, an electric power and an efficiency of Examples 1-1 to 1-3 are improved, compared to those of Examples A and B.

## Claims

1. A condensed-cyclic compound represented by one of Compound 1-1, Compound 1-2, and/or Compound 1-3, wherein:
Compound 1-1 is: 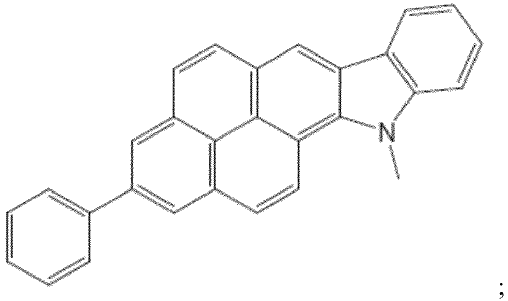
Compound 1-2 is: 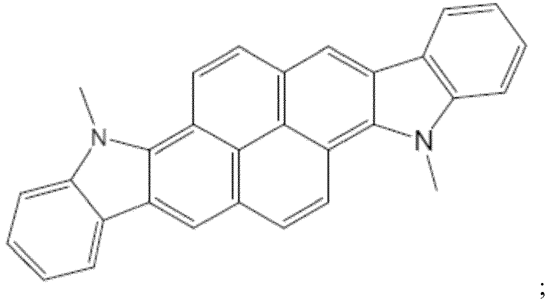
Compound 1-3 is:

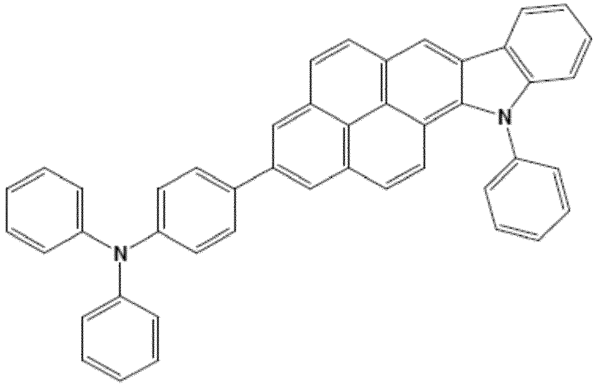

2. The condensed-cyclic compound of claim 1, wherein the condensed cyclic compound is Compound 1-1:

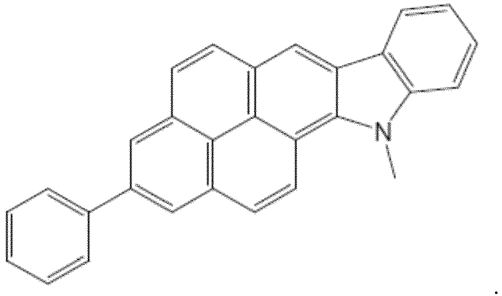

3. The condensed-cyclic compound of claim 1, wherein the condensed cyclic compound is Compound 1-2:

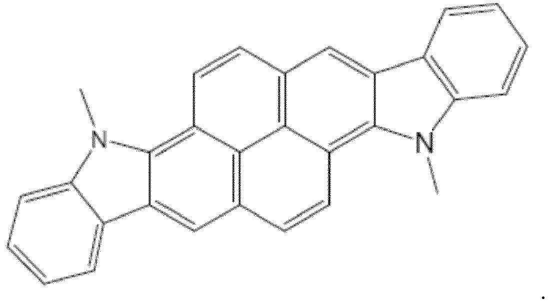

4. The condensed-cyclic compound of claim 1, wherein the condensed cyclic compound is Compound 1-3:

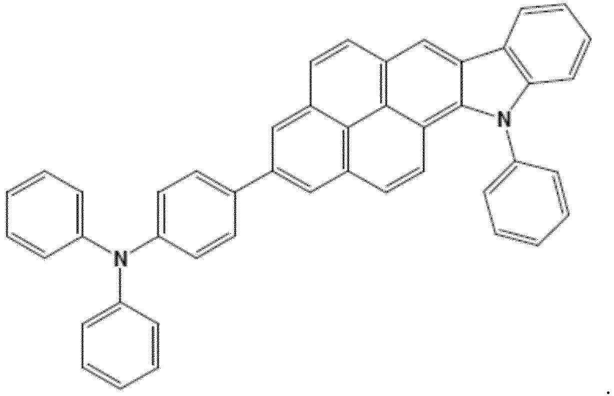

5. An organic light emitting diode comprising: a first electrode, a second electrode facing the first electrode, and an organic layer interposed between the first electrode and the second electrode, wherein the organic layer comprises a condensed-cyclic compound of one of claims 1 to 4.

6. The organic light emitting diode of claim 5, wherein the organic layer comprises a light emitting layer.

## Patentansprüche

1. Eine kondensiert-cyclische Verbindung, die durch eine der Verbindung 1-1, Verbindung 1-2 und/oder Verbindung 1-3 dargestellt ist, wobei:
Verbindung 1-1 ist: 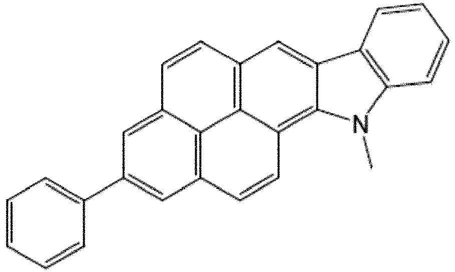
Verbindung 1-2 ist: 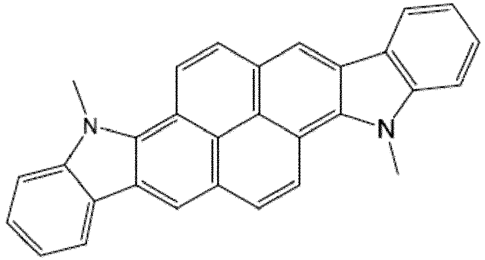
Verbindung 1-3 ist:

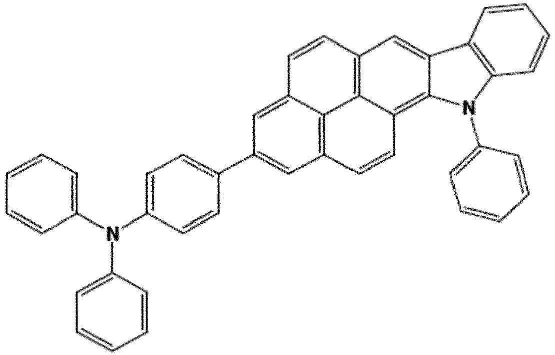

2. Die kondensiert-cyclische Verbindung nach Anspruch 1, wobei die kondensiert-cyclische Verbindung Verbindung 1-1 ist:

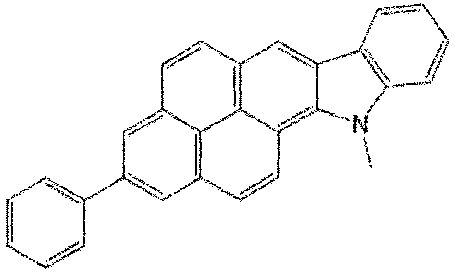

3. Die kondensiert-cyclische Verbindung nach Anspruch 1, wobei die kondensiert-cyclische Verbindung Verbindung 1-2 ist:

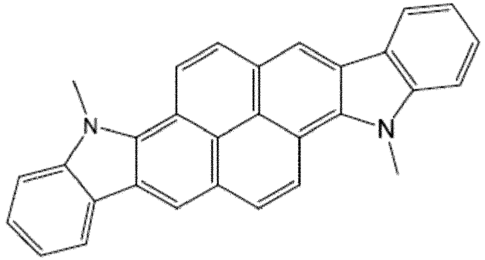

4. Die kondensiert-cyclische Verbindung nach Anspruch 1, wobei die kondensiert-cyclische Verbindung Verbindung 1-3 ist:

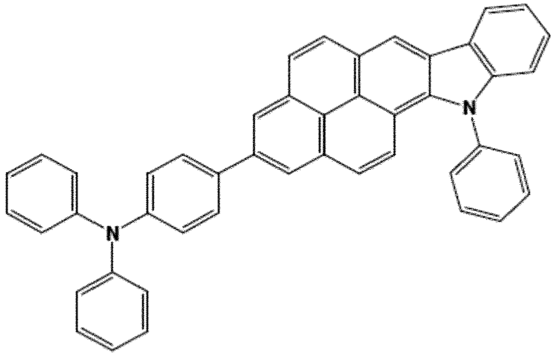

5. Eine organische lichtemittierende Diode, aufweisend: eine erste Elektrode, eine zweite Elektrode, die der ersten Elektrode zugewandt ist, und eine organische Schicht, die zwischen die erste Elektrode und die zweite Elektrode eingefügt ist, wobei die organische Schicht eine kondensiert-cyclische Verbindung nach einem der Ansprüche 1 bis 4 aufweist.

6. Die organische lichtemittierende Diode nach Anspruch 5, wobei die organische Schicht eine lichtemittierende Schicht aufweist.

## Revendications

1. Composé cyclique condensé représenté par l'un parmi le composé 1-1, le composé 1-2 et/ou le composé 1-3, dans lequel :
le composé 1-1 est : 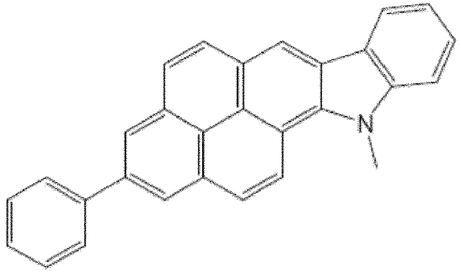
le composé 1-2 est : 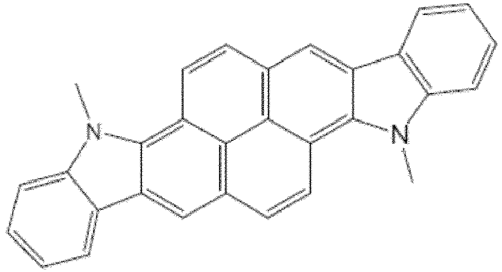
le composé 1-3 est :

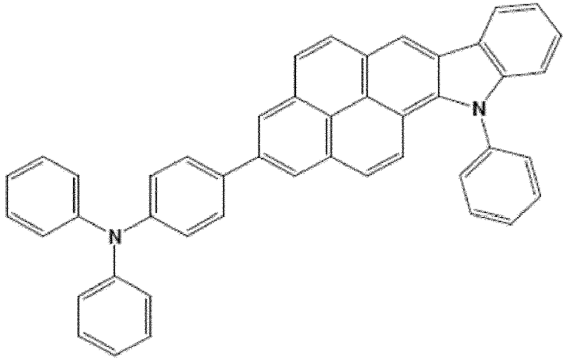

2. Composé cyclique condensé selon la revendication 1, lequel composé cyclique condensé est le composé 1-1 :

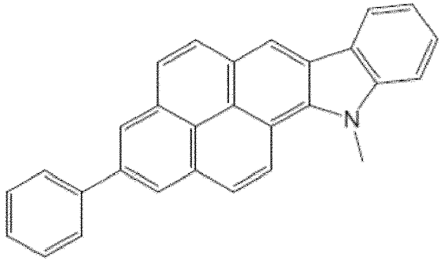

3. Composé cyclique condensé selon la revendication 1, lequel composé cyclique condensé est le composé 1-2 :

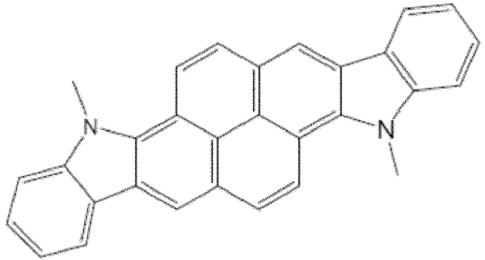

4. Composé cyclique condensé selon la revendication 1, lequel composé cyclique condensé est le composé 1-3 :

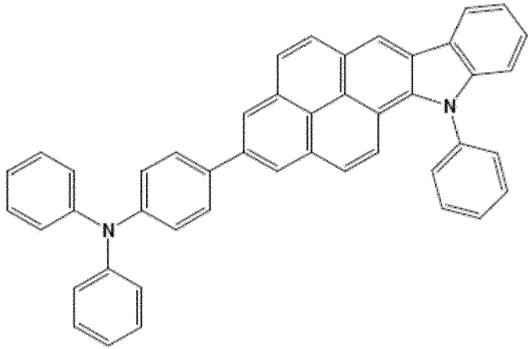

5. Diode luminescente organique comprenant : une première électrode, une deuxième électrode faisant face à la première électrode, et une couche organique interposée entre la première électrode et la deuxième électrode, dans laquelle la couche organique comprend un composé cyclique condensé selon l'une des revendications 1 à 4.

6. Diode luminescente organique selon la revendication 5, dans laquelle la couche organique comprend une couche luminescente.
